# EUROPÄISCHE PATENTANMELDUNG

(11) **EP 2 910 921 A1**
(43) Veröffentlichungstag der Anmeldung: **26.08.2015**
(21) Anmeldenummer: 15154652.0
(22) Anmeldetag: 11.02.2015
(51) Int. Cl.: G01N 1/40, B01L 7/00, F25B 21/02, F25B 21/04, G01N 1/42, G01N 1/44, H01L 35/32, G01N 3/54, G01N 11/00, G01N 35/00, G01N 25/14

(54) **Verfahren zur Einstellung der Temperatur und Temperierbehälter**

(30) Priorität: 20.02.2014 AT 501272014
(71) Anmelder: Anton Paar ProveTec GmbH, 15827 Blankenfelde-Mahlow (DE)
(72) Erfinder: Kindt, Carsten, 12247 Berlin (DE); Eilers, Helmut, 15732 Schulzendorf (DE)
(74) Vertreter: Wildhack & Jellinek

(57) **Zusammenfassung**

Die Erfindung betrifft ein Verfahren zur Einstellung der Temperatur in einem zur Aufnahme einer Probe ausgerüsteten Temperierbehälter (2) unter Einsatz zumindest eines unter Sicherstellung eines Wärmeübergangskontaktes an der Außenwandfläche des Behälters (2) angebrachten Peltier-Elementes (3).

Erfindungsgemäß ist unter anderem vorgesehen,
- dass an der dem Behälter (2) abgewandten Fläche des Peltier-Elementes (3) unter Sicherstellung eines Wärmeübergangskontaktes ein Speicherblock (4) aus metallischem Material angebracht wird,
- dass an der dem Peltier-Element (3) abgewandten Rückfläche des Speicherblockes (4) ein weiteres Peltier-Element (4) oder eine Peltier-Element-Kaskade unter Sicherstellung eines Wärmeübergangskontaktes angebracht wird,
- dass zur Veränderung des Temperaturniveaus des Behälters (2) auf eine angestrebte Zieltemperatur hin oder in Richtung dieser Zieltemperatur der Speicherblock (4) mit dem weiteren Peltier-Element (5) oder der Peltier-Element-Kaskade in Richtung dieser Zieltemperatur hin oder darüber hinaus erwärmt oder abgekühlt wird.

## Beschreibung

Die Erfindung betrifft ein Verfahren zur Einstellung der Temperatur in einem Temperierbehälter gemäß dem Oberbegriff des Patentanspruches 1. Des weiteren betrifft die Erfindung einen Temperierbehälter, insbesondere zur Durchführung dieses Verfahrens.

Derartige Temperierbehälter können unterschiedlichen Aufbau bzw. Form, z.B. rechteckigen oder runden Umfang, aufweisen und für unterschiedliche Zwecke vorgesehen sein. Insbesondere ist es auch möglich, mit derartigen Temperierbehältern normgerechte Untersuchungs- bzw. Messbedingungen, insbesondere für Erdölerzeugnisse, einzuhalten. Es geht hier vor allem um normgerechte Untersuchungsbedingungen, beispielsweise für die Messung des CFPP (Cold Filter Plugging Point), der die Grenze der Filtrierbarkeit von Erdölprodukten bestimmt.

Der Begriff Filtrierbarkeitsgrenze beziehungsweise Cold Filter Plugging Point des Temperaturgrenzwertes der Filtrierbarkeit bezeichnet eine Kälteeigenschaft von Dieselkraftstoffen und Heizöl EL (Extra Leicht). Dies ist die Temperatur in Grad Celsius, bei der ein Prüffilter unter definierten Bedingungen durch ausgefallene (n-)Paraffine verstopft. Bei dieser Methode wird die Probe mit einer konstanten Rate abgekühlt und dabei in definierten Abständen durch einen Prüffilter befördert. Vor Erreichen der Filtrierbarkeitsgrenze bilden sich beim sogenannten Cloud Point (CP) bereits Kristalle, die jedoch noch durch den Filter passen. Wenn die Kristalle zu groß werden, verstopft der Filter. Untersuchte Größen sind der Cloud Point, bei dem die Trübung der Probe durch Nebelwolken der ausfallende Paraffine etc. optisch untersucht und zu definierten Zeitpunkten durchstrahlt wird, und der Pour Point- Test, bei dem eine Untersuchung der Fließfähigkeit der temperierten Probe erfolgt. Aber auch andere Untersuchungen von temperierten Proben hinsichtlich ihrer physikalischen Eigenschaften wie Viskosität, rheologischen Eigenschaften, etc. sind unter Einsatz des erfindungsgemäßen Verfahrens und der Vorrichtung denkbar.

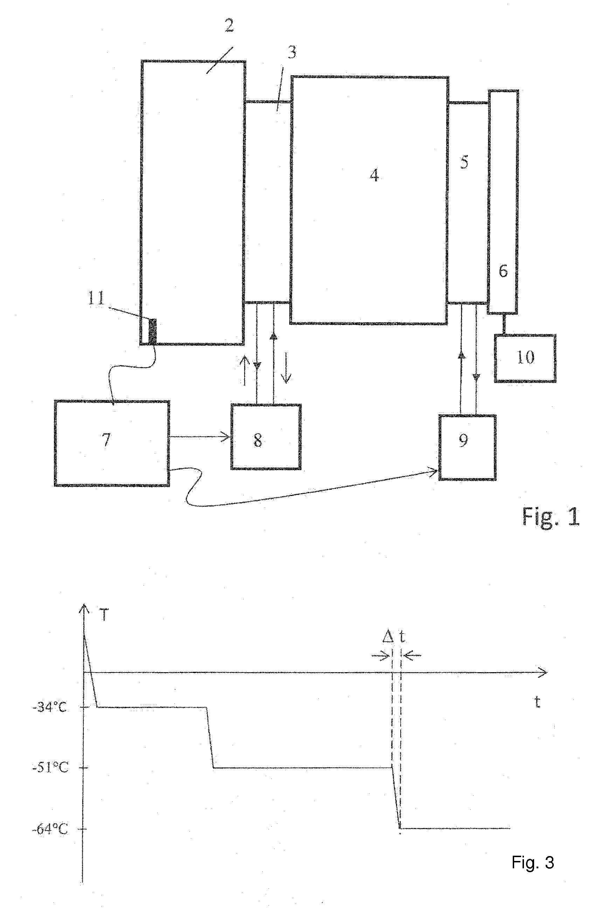

Derartigen Messverfahren liegen zumeist genaue Normvorschriften zugrunde, die einzuhaltende Temperaturprofile vorgeben. Für die CFPP von Diesel beispielsweise sind dies die EN116, ASTM D 6371 und DIN EN 16329. Diese Normen schreiben genaue Temperaturprofile für einen Temperierbehälter, in diesem Fall eine Kühlkammer vor, in die ein Glasbehälter im Abstand zur Innenwand der Kühlkammer eingesetzt wird, welcher Glasbehälter die zu untersuchende Probe enthält. Auf den Glasbehälter wird ein Deckel dicht aufgesetzt, der über eine Durchleitung im Deckel eine definierte Probenmenge mittels einer Vakuumpumpe bis zu einer Marke in ein Testvolumen überführt. Für den CFPP -Test ist im Probenweg zum Testvolumen ein Sieb mit definierter Maschenweite montiert, durch das die Probe gepumpt wird. Die Temperatur der Probe wird gemessen und bei jeder Temperaturabnahme um ein Grad eine Entnahme des die Probe bildenden Treibstoffes mittels der Pumpe versucht. Bei tieferen Temperaturen beginnt das Paraffin im Diesel auszuflocken, Schwebeteilchen setzen sich im Filtersieb ab und verstopfen dieses. Wenn dadurch die Füllzeit für das Testvolumen eine bestimmte Zeitspanne übersteigt oder der Treibstoff nicht mehr vollständig aus dem Testvolumen in den Glasbehälter zurückläuft, wird dies als die Filtrierbarkeitsgrenze gemäß CFPP angesehen. Normgemäß ist dabei vorgesehen, dass die Temperatur in der Temperierkammer vorab auf -34°C eingestellt und die Messung der Probe erfolgt, dass nach den Messungen bei dieser Temperatur der Behälter auf - 51°C abgekühlt wird und auf dieser Temperatur gemessen wird und dass schließlich eine Abkühlung und Messung bei -67°C erfolgt. Die Abkühlung hat dabei - wie vorgeschrieben - rasch zu erfolgen. Es müssen sehr enge Zeitvorgaben für den Abkühlprozess des Temperierbehälters eingehalten werden, und dementsprechend muss die Kühlleistung ausreichend sein, um z.B. innerhalb von 150 sec den den Probenbehälter umschließenden Temperierbehälter in einer vorgegebenen Zeitspanne um z.B. 17 Grad abzukühlen. Typische Kühlprofile für eine solche Abkühlung haben die in Fig. 3 dargestellte Form, wobei die Temperatur T über die Zeit t aufgetragen ist. Es ist ohne weiteres möglich, andere Abkühlungsverläufe oder Erwärmungen mit dem erfindungsgemäßen Temperierbehälter auszuführen.

Die Peltiertechnik konkurriert mit den herkömmlichen Kompressor- und Absorber-Kühlsystemen. Wenn man die Kosten der Kälteleistung dieser Kühlsysteme vergleicht, so kommt man zu dem Ergebnis, dass Peltier-Elemente teurer sind. Die Vorzüge der Peltiertechnik dürfen aber nicht übersehen werden. Bei Anwendungen, die nur geringe Kälteleistungen erfordern, benötigen Peltier-Elemente wesentlich weniger Platz. Die Regelung der Leistung eines Peltierlelements über den Betriebsstrom ist einfach und genau. Durch einfaches Umpolen der Stromrichtung lassen sich Peltier-Elemente sowohl zum Heizen als auch zum Kühlen verwenden. Weiterer Vorteil einer Peltierkühlung ist eine preiswerte externe Kühlversorgung für die Gegenkühlung der Elemente.

Aufgrund der verhältnismäßig geringen Kühlleistung der Peltierelemente sind jedoch hohe dynamische Leistungsanforderungen wie für Temperierbehälter, die insbesondere zur Erfüllung der oben beschriebene Normen benötigt werden, zumeist nicht erfüllbar. Soferne große Temperaturunterschiede in kürzester Zeit überwunden werden müssen, ist ein Peltierelement aufgrund seiner limitierten Kühlleistung bei hohen Temperaturunterschieden nicht einsetzbar.

Es existieren natürlich ein- und mehrstufige Anordnungen mit Peltierelementen, die die Leistung erhöhen sollten, z.B. Peltierelemente, die übereinander montiert sind, als auch Elemente in mehrstufiger Ausführung. Peltierelemente sind allerdings für rasche Kühlschritte, so wie sie normgemäß bei Geräten zur Erdölprüfung gefordert werden, nicht geeignet. Derartige Kühlungen erfolgen mit einem Stirling- oder Kompressorkühler. Kompressorkühler müssen dazu aber mindestens zweistufig ausgeführt sein, was sie extrem teuer und groß macht. Ähnliches gilt für Stirlingkühler.

Hauptaufgabe der Erfindung ist es, für einen Probenbehälter einen Temperierbehälter, insbesondere für normgemäße Messverfahren, zur Verfügung zu stellen, der klein und leicht ist und exakt arbeitet und eine rasche Temperaturänderung ermöglicht. Insbesondere soll der Temperierbehälter bzw. sein Probenaufnahmeraum rasch auf bestimmte Temperaturniveaus abzukühlen sein und soll die erreichte Temperatur konstant halten können, während die Probe im Probenglas bzw. einem Messbehälter über Wärmekonvektion und/oder -strahlung die Temperatur des Temperierbehälters annimmt.

Erfindungsgemäß ist ein Verfahren der eingangs genannten Art mit den im Kennzeichen des Anspruchs 1 angegebenen Merkmalen charakterisiert. Es ist erfindungsgemäß somit vorgesehen,
- dass an der dem Behälter abgewandten Fläche des Peltier-Elementes unter Sicherstellung eines Wärmeübergangskontaktes ein Speicherblock aus metallischem Material angebracht wird,
- dass an der dem Peltier-Element abgewandten Rückfläche des Speicherblockes ein weiteres Peltier-Element oder eine Peltier-Element-Kaskade unter Sicherstellung eines Wärmeübergangskontaktes angebracht wird,
- dass zur Veränderung des Temperaturniveaus des Behälters auf eine angestrebte Zieltemperatur hin oder in Richtung dieser Zieltemperatur der Speicherblock mit dem weiteren Peltier-Element oder der Peltier-Element-Kaskade in Richtung dieser Zieltemperatur hin oder darüber hinaus erwärmt oder abgekühlt wird und
- dass für die Realisierung einer raschen Temperaturänderung im Behälter die Stromdurchflussrichtung des Peltier-Elementes derart geschaltet wird, dass dieses den Behälter in Richtung auf die angestrebte Zieltemperatur hin erwärmt oder abkühlt und damit gleichzeitig den Übergang der im Speicherblock enthaltenen Wärmemenge oder Kältemenge durch sich selbst hindurch auf den Behälter zur Verstärkung der vom Peltier-Element erfolgenden Erwärmung oder Abkühlung freigibt.

Eine derartige Vorgangsweise ist insbesondere für die Durchführungen von Messungen an Erdölprodukten, vorzugsweise zur Ermittlung der Filtrierbarkeit von Dieselkraftstoffen und Haushaltheizölen zweckmäßig. Insbesondere sind mit einem derartigen Verfahren die Vorschriften gemäß DIN EN 116 erfüllbar.

Die Erfindung benutzt die Möglichkeit, Wärme- bzw. Kälteenergie mit Hilfe von Peltier-Elementen zu speichern und bei Bedarf abzugeben. Wesentlich dazu ist es, dass sich zwischen dem Peltier-Element und dem weiteren Peltier-Element der Speicherblock aus Metall befindet. Dieser Metallblock ist so bemessen, dass er eine ausreichende Menge an Wärmeenergie für die geforderte Anwendung speichern und diese Wärmemenge ausreichend rasch aufnehmen und abgeben kann. Als Material für eine derartigen Wärme- bzw. Kältespeicher sind Materialien mit hoher spezifischer Wärmekapazität und Wärmeleitfähigkeit, beispielsweise Aluminium, Kupfer, Messing, Gold, Silber, Magnesium, usw. gut geeignet.

Es wird in diesem Zusammenhang bemerkt, dass mit dem erfindungsgemäßen Verfahren und der erfindungsgemäßen Vorrichtung sowohl hohe Kühlraten als auch Erwärmungsraten erreichbar sind. Besondere Vorteile ergeben sich jedoch beim Abkühlen der Temperatur eines eine Probe aufnehmenden Temperierbehälters.

Um bei der erfindungsgemäßen Vorgangsweise die Temperatur im Temperierbehälter nicht oder gezielt zu beeinflussen, kann vorgesehen sein, dass während des Aufheizens oder Abkühlens des Speicherblockes das Temperaturniveau im Behälter mit dem Peltier-Element eingeregelt und damit vorzugsweise konstant gehalten oder kontinuierlich verändert wird.

Um eine ausreichend rasche und zuverlässige Temperaturänderung im Temperierbehälter zu erreichen, ist erfindungsgemäß vorgesehen, dass die Erwärmung oder Abkühlung des Speicherblockes über oder unter die angestrebte Zieltemperatur in einem Ausmaß erfolgt, dass die im Speicherblock enthaltene und an den Behälter über das Peltier-Element abführbare Wärme- oder Kältemenge ausreicht, die angestrebte Zieltemperatur des Behälters, vorzugsweise innerhalb einer vorgegebenen Zeitspanne, zu erreichen. Die erforderliche Bemessung der Wärme- oder Kältemenge ist einfach durch Berücksichtigung der abzukühlenden Massen und deren spezifischer Wärme möglich, wobei gleichzeitig allfällige Umgebungseinflüsse sowie Wärmekonvektion und Wärmeabstrahlung berücksichtigt werden. Für den Fachmann stellt es keine Schwierigkeit dar, die erforderlichen Materialien und Dimensionierungen des Speicherblockes und des Temperierbehälters zu wählen, um einen raschen Übergang ausreichender Wärmemengen zu erreichen.

Um allen Eventualitäten gerecht zu werden, ist vorgesehen, dass die Speicherkapazität des Speicherblockes für die Speicherung von Wärme- oder Kältemengen größer bemessen wird als die für eine gewünschte Temperaturänderung des Behälter erforderliche Wärme- oder Kältemenge. Durch entsprechende Ansteuerung des Peltier-Elementes kann der Wärmeübergang vom Speicherblock zum Temperierbehälter bei Erreichen einer vorgegebenen Zieltemperatur unterbrochen werden und dann mit diesem Peltier-Element die Zieltemperatur exakt eingeregelt bzw. der Temperierbehälter auf dieser Temperatur gehalten werden oder die erreiche Temperatur verändert werden.

Um ausreichende Wärme- oder Kältemengen zur Verfügung zu haben, ist es zweckmäßig, wenn bei einer Abkühlung des Behälters auf die oder in Richtung der gewünschte(n) Zieltemperatur die Temperatur des Speicherblocks auf einen tieferen Wert als die Zieltemperatur abgesenkt wird und zur Abkühlung des Behälters das Peltier-Element derart mit Strom durchflossen wird, dass es den Behälter kühlt und das Durchströmen der im Speicherblock enthaltenen Kältemengen ermöglicht.

Je nach Bedarf ist es möglich, dass nach Erreichen der angestrebten Zieltemperatur im Behälter diese Zieltemperatur mit dem Peltier-Element für eine vorgegebene Zeitspanne konstant gehalten oder kontinuierlich verändert wird und danach zumindest eine, vorzugsweise sprunghafte oder lineare Erwärmung oder Abkühlung auf eine angestrebte Zieltemperatur vorgenommen wird.

Für einen effizienten Betrieb des Peltier-Elementes und des weiteren Peltier-Elementes und eine rasche Abkühlung des Speicherblockes ist es von Vorteil, wenn das Peltier-Element bzw. die Peltier-Element-Kaskade an ihrer dem Behälter abgewandten Rückfläche, vorzugsweise abhängig von der gewünschten Temperatureinstellung im Speicherblock, beheizt oder gekühlt wird. Unterstützt wird diese Vorgangsweise, wenn bei einer Abkühlung des Behälters das Peltier-Element auf volle Leistung geschaltet wird und kurz vor oder bei Erreichen der Zieltemperatur in einem Regelmodus zur Konstanthaltung der Temperatur des Behälters geschaltet wird.

Erfindungsgemäß ist vorgesehen, dass die Einstellung der Temperatur für einen Temperierbehälter vorgenommen wird, in dem eine Messeinheit zur Bestimmung von temperaturabhängigen Materialparametern, insbesondere der viskosen Eigenschaften von Proben, vorzugsweise von Erdölerzeugnissen, angeordnet wird, welche Messeinheit temperiert oder für die Messungen auf einer angestrebten Zieltemperatur gehalten wird.

Ein erfindungsgemäßer Temperierbehälter ist dadurch gekennzeichnet,
- dass an der dem Behälter abgewandten Fläche des Peltier-Elementes unter Sicherstellung eines Wärmeübergangskontaktes ein Speicherblock aus metallischem Material angebracht ist,
- dass an der dem Peltier-Element abgewandten Rückfläche des Speicherblockes unter Sicherstellung eines Wärmeübergangskontaktes ein weiteres Peltier-Element oder eine Peltier-Element-Kaskade angebracht ist,
- dass zur Veränderung des Temperaturniveaus des Behälters auf eine angestrebte Zieltemperatur hin oder in Richtung dieser Zieltemperatur eine Steuereinheit für den Stromdurchfluss durch das Peltier-Element und das weitere Peltier-Element oder die Peltier-Element-Kaskade vorgesehen ist, mit welcher Steuereinheit der Speicherblock mit dem weiteren Peltier-Element oder der Peltier-Element-Kaskade in Richtung dieser Zieltemperatur oder darüber hinaus erwärmbar oder abkühlbar ist und
- dass die Steuereinheit für die Realisierung einer raschen Temperaturänderung im Behälter bzw. dessen Probenaufnahmeraum die Stromdurchflussrichtung des Peltier-Elementes derart schaltet, dass dieses den Behälter in Richtung auf die angestrebte Zieltemperatur erwärmt oder abkühlt und damit gleichzeitig den Übergang der im Speicherblock enthaltenen Wärme- oder Kältemenge auf den Behälter zur Verstärkung der vom Peltier-Element erfolgenden Erwärmung oder Abkühlung freigibt.

Aufgrund der Anordnung des Speicherblockes zwischen dem mit der Behälteraußenwand in Wärmekontakt stehenden Peltier-Element und dem weiteren Peltier-Element an der dem Behälter abgewandten Rückseite des Speicherblockes wird es einfach möglich, den Speicherblock rasch abzukühlen und ausreichend rasch eine Wärmemenge bzw. Kältemenge zur Verfügung zu stellen, die ausreicht, um den Temperierbehälter innerhalb einer vorgegebenen Zeitspanne auf eine gewünschte Temperatur abzukühlen. Diese Temperatur kann dem an der Behälteraußenwand angeordneten Peltier-Element konstant gehalten oder in vorgegebener Weise verändert werden.

Es ist möglich, um die Außenwandfläche des Temperierbehälters herum eine Mehrzahl von Peltier-Elementen anzuordnen. Vorteilhafterweise werden zwei bis vier Peltier-Elemente vorgesehen, die in gleichen Abständen längs des Behälterumfanges angeordnet sind. Jedes dieser Peltier-Elemente ist mit einem eigenen Speicherblock versehen. Prinzipiell könnte auch ein durchgehender Speicherblock in Form eines Speicherringes vorgesehen werden. Vorzugsweise ist jedem Peltier-Element ein weiteres Peltier-Element an der dem Behälter abgewandten Rückfläche des Speicherblockes zugeordnet. Bei Einsatz eines ringförmigen Speicherblockes kann auch eine die Anzahl der Peltier-Elemente übersteigende Anzahl von weiteren Peltier-Elementen oder Peltierelement-Kaskaden vorgesehen sein. Wesentlich ist, dass die weiteren Peltier-Elemente den Speicherblock innerhalb einer gewünschten Zeitspanne ausreichend tief abkühlen oder erwärmen können.

Um die Funktion der weiteren Peltier-Elemente zu unterstützen, kann vorgesehen sein, dass das Peltier-Element oder die Peltier-Element-Kaskade an ihrer behälterfernen Fläche mit einem Wärmetauscher versehen sind, mit dem die Peltier-Elemente oder die Peltier-Element-Kaskade erwärmbar oder abkühlbar ist.

Die Peltier-Elemente sind an der Außenwandfläche des Behälters angeschweißt, angelötet, angeklebt oder auf andere Art befestigt. Bei eine gekrümmten Außenfläche besitzenden Behältern ist Sorge dafür zu tragen, dass die flächigen Peltier-Elemente in gutem Wärmeübergangskontakt mit der Behälterwand stehen. Das kann z.B. durch eben ausgeführte Bereiche der Behälterwand oder durch Einsetzen von wärmeleitenden Zwischenstücken oder Füllmaterialien in vorhandene Zwischenräume erfolgen.

Für den Betrieb ist es zweckmäßig, wenn die Steuereinheit während des Aufheizens oder des Abkühlens des Speicherblockes das Temperaturniveau im Behälter mit dem Peltier-Element eingeregelt und gegebenenfalls konstant hält oder kontinuierlich verändert.

Für die Temperatureinstellung ist es zweckmäßig, wenn im Inneren des Behälters bzw. des Probenaufnahmeraumes eine Temperaturmesseinheit angeordnet ist, die an die Steuereinheit angeschlossen ist.

Die Steuereinheit steuert die Peltier-Elemente zu entsprechenden Zeitpunkten an und misst gleichzeitig die Temperatur im Inneren des Temperierbehälters. Abhängig von der gemessenen Temperatur erfolgt die Steuerung des die Peltier-Elemente durchströmenden Gleichstroms bzw. die Umpolung bzw. Umkehr der Stromrichtung, mit der die Peltier-Elemente durchströmt sind.

Erfindungsgemäß ist es möglich, während des Aufheizens oder Abkühlens des Speicherblockes das Temperaturniveau im Behälter mit dem Peltier-Element einzuregeln und die am Beginn einer solchen Rampe überschüssige Energie im Speicherblock 4 zu sammeln und später zur Erreichung der Zieltemperatur in tieferen Temperaturbereichen zur Verfügung zu stellen. Damit wird die Rampe energieoptimiert betrieben und es werden extremere Zieltemperaturen möglich.

Der erfindungsgemäße Temperierbehälter eignet sich besonders gut zur Temperierung von Messeinheiten zu Bestimmung der viskosen Eigenschaften von Proben, vorzugsweise von Erdölerzeugnissen bzw. zur Abkühlung von Temperierbehältern, in denen derartige Messeinheiten eingesetzt sind und auf einer bestimmten Temperatur gehalten und von dieser Temperatur in Richtung auf eine vorgegebene Zieltemperatur abgekühlt oder erwärmt werden sollen.

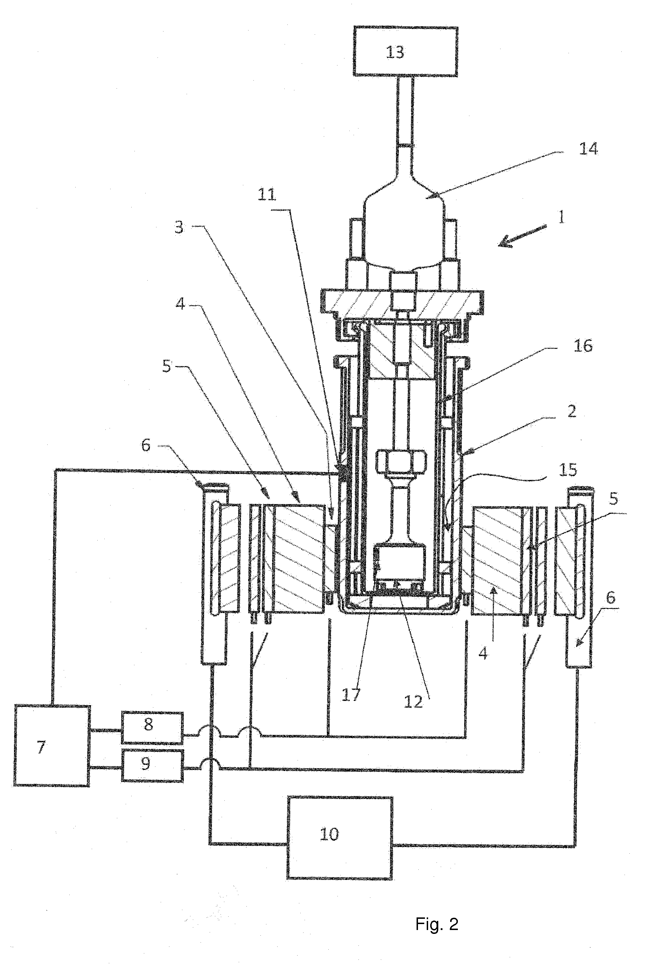

Im Folgenden wir die Erfindung beispielsweise anhand der Zeichnungen näher erläutert. Es zeigen Fig. 1 einen prinzipiellen Aufbau einer erfindungsgemäßen Vorrichtung. Fig. 2 zeigt schematisch eine in dem Temperierbehälter eingesetzte Messeinheit, so wie sie für die CFPP-Messung von Erdölprodukten, insbesondere Diesel, eingesetzt wird. An die Außenwandfläche eines Temperierbehälters 2 ist ein Peltier-Element 3 mit Wärmeübertragungskontakt angeschlossen. Dieses Peltier-Element 3 kann mit einem Gegenkühler 6 ausgerüstet sein. An der dem Behälter 2 abgewandten Fläche des Peltier-Elementes 3 ist ein Speicherblock 4 befestigt. An der behälterfernen Rückfläche des Speicherblockes 4 ist ein weiteres Peltier-Element 5 oder eine Peltierelement-Kaskade angeschlossen, welches Peltier-Element 5 mit einer Gegenkühleinheit 6 ausgestattet ist, die von einer Fluidkühl- oder Wärmeeinheit 10 mit Fluid versorgt wird. Für das Peltier-Element 3 kann der Speicherblock 4 die Funktion des Gegenkühlers ausüben.

Im Inneren des Temperierbehälters 2 bzw. seines Probenaufnahmeraumes befindet sich eine Temperaturmesseinheit 11, deren Ausgangssignal an eine Steuereinheit 7 angelegt ist. Die Steuereinheit 7 steuert Stromregler 8 und 9, wobei der Stromregler 8 die Stromrichtung und die Stromstärke des das Peltier-Element 3 durchströmenden Gleichstroms regelt. Die Regeleinheit 9 steuert die Stromstärke und die Durchflussrichtung des Gleichstroms durch das weitere Peltier-Element 5.

Der Querschnitt des Temperierbehälters 2 kann beliebig gewählt werden bzw. ist an die äußere Form der in den Behälter 2 eingesetzten Messeinheit 1 angepasst. Der Speicherblock 4 ist in der Regel von einem quaderförmigen Metallblock gebildet.

Das Peltier-Element 3 kann z. B. ständig eingeschaltet sein, um den Speicherblock 4 zu kühlen. Gleichzeitig kann das weitere Peltier-Element 5 so geschaltet werden, dass es auch den Speicherblock 4 kühlt und somit ebenfalls die Temperatur im Probenaufnahmeraum des Behälters 2 absenkt. Das Peltier-Element 3 kann auch gerade soviel Wärme oder Kälte erzeugen, wie nötig ist, um den Behälter 2 auf einer konstanten Temperatur zu halten und den Speicherblock 4 möglichst weit abzukühlen, wozu das Peltierelement 3 eigentlich in Heizschaltung betrieben wird und der Speicherblock 4 als Gegenkühlung fungiert.

Wird allerdings ein großer Temperatursprung benötigt, so wird das behälternahe Peltier-Element 3 in umgekehrter Richtung mit Strom durchflossen bzw. geschaltet, so dass die Kältemenge des auf einer tieferen Temperatur liegenden Speicherblockes 4 in den Behälter 2 leitet und zusätzlich den Behälter 2 noch aktiv kühlt. So ergibt sich die Möglichkeit, den Temperierkörper mit hoher Dynamik zu temperieren. Da die Temperatur des Behälters 2 mittels eines einzigen Peltier-Elements, das heißt dem Peltierelement 3, regelbar ist, ist die Regelung sehr präzise möglich.

Auf diese Weise werden ohne weiteres Kühlraten von 20 Grad in 150 Sekunden zur Verfügung gestellt.

Startet man eine Vermessung einer Probe beispielsweise bei Raumtemperatur, so beginnt das bevorzugterweise stärker dimensionierte weitere Peltier-Element 5 mit voller Leistung zu kühlen. Der Speicherblock 4 kühlt sofort ab. Wird eine Messung im Behälter 2 bei Raumtemperatur gewünscht, wird das behälternahe Peltier-Element 3 hauptsächlich im Heizmodus betrieben werden und die Steuer- und Regeleinheit 7 regelt die Wärmezu- und Abfuhr zum bzw. vom Behälter 2 gemäß der mit dem zumindest einen Temperaturmessfühler 11 ermittelten Temperatur im Behälter 2. Um die Temperatur rasch und genau regeln zu können, können auch mehrere Temperatursensoren im Behälter 2 eingebaut sein.

Im Kühlfall ist die Temperatur des Speicherblockes 4 vor Entnahme der Kältemenge wesentlich kleiner als die Temperatur im Behälter 2.

Zur Konstanthaltung der Temperatur des Behälters 2 wird das Peltierelement 3 in den Heizmodus geschaltet und temperiert den Behälter 2 und schützt diesen gleichzeitig gegen die im Speicherblock 4 vorherrschende Temperatur, die andernfalls über das Peltier-Element 3 den Behälter 2 kühlen würde.

Bei einer einfachen Ausführung der Erfindung kann das Peltier-Element 5 immer mit demselben, bevorzugterweise maximalen, Betriebsstrom betrieben werden und wird von der Steuer - und Regeleinheit 7 nur zu Beginn und Ende der Messung ein - und ausgeschaltet.

Verwendet man Peltierelement-Kaskaden um zu tieferen Temperaturen zu gelangen, können diese je nach der gewünschten Temperatur zu- oder abgeschaltet werden. Temperatursprünge nahe der Raumtemperatur und auch das Konstanthalten des Probenaufnahmeraumes 15 des Behälters 2 bei hohen Temperaturen erfodern nur ein Peltierelement 5, wogegen zum Erreichen tieferer Temperaturen die Peltierelement-Kaskade zuzuschalten ist.

Ist ein Temperatursprung zu tieferen Temperaturen zu realisieren, so wird das behälterseitige Peltierelement 3 in den Kühlmodus auf volle Leistung mit maximalem Betriebsstrom geschaltet und die Temperatur des Speicherblocks 4 steht zur Temperierung des Probeaufnahmeraumes 15 zur Verfügung. Spätestens ab Erreichen der gewünschten Zieltemperatur Probenaufnahmeraum 15 geht das Peltier-Element 3 wieder in den Regelmodus und der Betriebsstrom wird in Abhängigkeit von der erreichten Zieltemperatur geregelt.

Bevorzugt wird ein Regelalgorithmus gewählt, der bereits vor dem Erreichen der Zieltemperatur im Behälter 2 die Kühlung verringert, um ein starkes "Übersteuern" der Temperaturwerte im Probenaufnahmeraum 15 zu verhindern.

Da Peltier-Elemente durch Umpolen der Stromrichtung auch als Heizelemente verwendet werden können, ist der Betrieb der Anordnung prinzipiell auch als Heizeinrichtung möglich.

Fig. 2 zeigt den prinzipiellen Messaufbau für die Bestimmung der Filtrierbarkeitsgrenze von Dieselkraftstoffen oder Heizöl. Bei dieser Versuchsanordnung wird in den Probenaufnahmeraum 15 des Temperierbehälters 2 eine Messeinheit 1 eingesetzt, deren Aufbau durch die entsprechenden Normen EN 116, ASTM D 6371 oder DIN EN 16329 vorgegeben ist. In einen Glasbehälter 16 wird die zu bestimmende Probenflüssigkeit eingefüllt. Über ein Filter 12 wird mittels einer Pumpe 13, insbesondere Vakuumpumpe, die Flüssigkeit in ein Testvolumen 14 abgesaugt. Dieses Absaugen und Rückfließenlassen der Probenflüssigkeit bei unterschiedlichen Temperaturen aus bzw. in den Probenaufnahmeraum 15 wird so lange durchgeführt, bis ein Ansaugen oder ein Zurückfließen der Probeflüssigkeit durch ausfallende Paraffinteilchen nicht mehr in einer vorgegebenen Zeitspanne durchgeführt werden kann. Der wesentliche Teil des Messverfahrens ist dabei die Temperierung des Probenaufnahmeraumes 15 des Behälters 2 bzw. die Einstellung der Temperatur in der Messeinheit 1, das heißt der Temperatur der Probenflüssigkeit. Diese Einstellung wird mit der erfindungsgemäßen Temperiereinheit bzw. mit der erfindungsgemäßen Vorgangsweise optimal erreicht.

Fig. 3 zeigt einen für ein derartiges Messverfahren typischen Temperierverlauf. Die Temperierkammer 2 wird im Zuge der normgemäßen Messvorbereitung auf -34°C abgekühlt. Die Peltier-Elemente 3 und 5 kühlen dabei mit voller Leistung. Bei Erreichen dieser Zieltemperatur wird mit dem Peltier-Element 3 die Temperatur der Temperierkammer 2 konstant gehalten, während das Peltier-Element 5 das Speicherelement weiter kühlt. Nun werden der Glasbehälter 16 mit der zu untersuchenden Probe bzw. die gesamte Messeinheit 1 in den Probenaufnahmeraum 15 des Temperierbehälters 2 eingesetzt. Die Probentemperatur wird mit einer weiteren Temperaturmesseinheit 17 im Probenglas bzw. einem Filterhalter gemessen und gegebenenfalls an eine Steuer- und Auswerteeinheit 7 zur automatischen Versuchsdurchführung übermittelt. Die Messung der temperaturabhängigen Eigenschaft (CFPP) erfolgt nun normgemäß ab einer Probentemperatur von 20°C jedes Grad fallend einmal. In dieser Zeit wird die Temperatur des Temperierbehälters 2 mit dem Peltier-Element 3 weiter konstant gehalten und der Speicherblock 4 wird mit dem weiteren Peltier-Element 5 weiter abgekühlt. Wenn die Probentemperatur - 20°C erreicht hat, startet die rasche Abkühlung der Probenkammer auf - 51 °C. Die normgemäß vorgeschriebene Zeit für den Temperatursprung wird hier durch rasches Entleeren des Speicherblockes 4 erreicht. Weitere Temperautrampen und stufen können in vergleichbarer Weise umgesetzt werden.

## Patentansprüche

1. Verfahren zur Einstellung der Temperatur in einem zur Aufnahme einer Probe ausgerüsteten Temperierbehälter (2) unter Einsatz zumindest eines unter Sicherstellung eines Wärmeübergangskontaktes an der Außenwandfläche des Behälters (2) angebrachten Peltier-Elementes (3), **dadurch gekennzeichnet,**
- **dass** an der dem Behälter (2) abgewandten Fläche des Peltier-Elementes (3) unter Sicherstellung eines Wärmeübergangskontaktes ein Speicherblock (4) aus metallischem Material angebracht wird,
- **dass** an der dem Peltier-Element (3) abgewandten Rückfläche des Speicherblockes (4) ein weiteres Peltier-Element (4) oder eine Peltier-Element-Kaskade unter Sicherstellung eines Wärmeübergangskontaktes angebracht wird,
- **dass** zur Veränderung des Temperaturniveaus des Behälters (2) auf eine angestrebte Zieltemperatur hin oder in Richtung dieser Zieltemperatur der Speicherblock (4) mit dem weiteren Peltier-Element (5) oder der Peltier-Element-Kaskade in Richtung dieser Zieltemperatur hin oder darüber hinaus erwärmt oder abgekühlt wird und
- **dass** für die Realisierung einer raschen Temperaturänderung im Behälter (2) die Stromdurchflussrichtung des Peltier-Elementes (3) derart geschaltet wird, dass dieses den Behälter (2) in Richtung auf die angestrebte Zieltemperatur hin erwärmt oder abkühlt und damit gleichzeitig den Übergang der im Speicherblock (4) enthaltenen Wärmemenge oder Kältemenge durch sich selbst hindurch auf den Behälter (2) zur Verstärkung der vom Peltier-Element (3) erfolgenden Erwärmung oder Abkühlung freigibt.

2. Verfahren nach Anspruch 1, **dadurch gekennzeichnet, dass** während des Aufheizens oder Abkühlens des Speicherblockes (4) das Temperaturniveau im Behälter (2) mit dem Peltier-Element (3) eingeregelt und damit vorzugsweise konstant gehalten oder kontinuierlich verändert wird.

3. Verfahren nach Anspruch 1 oder 2, **dadurch gekennzeichnet, dass** die Erwärmung oder Abkühlung des Speicherblockes (4) über oder unter die angestrebte Zieltemperatur in einem Ausmaß erfolgt, dass die im Speicherblock (4) enthaltene und an den Behälter (2) über das Peltier-Element (3) abführbare Wärme- oder Kältemenge ausreicht, die angestrebte Zieltemperatur des Behälters (2), vorzugsweise innerhalb einer vorgegebenen Zeitspanne (Δ t), zu erreichen.

4. Verfahren nach einem der Ansprüche 1 bis 3, **dadurch gekennzeichnet, dass** die Speicherkapazität des Speicherblockes (4) für die Speicherung von Wärme- oder Kältemengen größer bemessen wird als die für eine gewünschte Temperaturänderung des Behälter (2) erforderliche Wärme- oder Kältemenge.

5. Verfahren nach einem der Ansprüche 1 bis 4, **dadurch gekennzeichnet, dass** bei einer Abkühlung des Behälters (2) auf die oder in Richtung der gewünschte(n) Zieltemperatur die Temperatur des Speicherblocks (4) auf einen tieferen Wert als die Zieltemperatur abgesenkt wird und zur Abkühlung des Behälters (2) das Peltier-Element (3) derart mit Strom durchflossen wird, dass es den Behälter (2) kühlt und das Durchströmen der im Speicherblock (4) enthaltenen Kältemengen ermöglicht.

6. Verfahren nach einem der Ansprüche 1 bis 5, **dadurch gekennzeichnet, dass** nach Erreichen der angestrebten Zieltemperatur im Behälter (2) diese Zieltemperatur mit dem Peltier-Element (3) für eine vorgegebene Zeitspanne konstant gehalten oder kontinuierlich verändert wird und danach zumindest eine, vorzugsweise sprunghafte oder lineare Erwärmung oder Abkühlung auf eine angestrebte Zieltemperatur vorgenommen wird.

7. Verfahren nach einem der Ansprüche 1 bis 6, **dadurch gekennzeichnet, dass** die Einstellung der Temperatur für einen Temperierbehälter (2) vorgenommen wird, in dem eine Messeinheit (1) zur Bestimmung von temperaturabhängigen Materialparametern, insbesondere der viskosen Eigenschaften von Proben, vorzugsweise von Erdölerzeugnissen, angeordnet wird, welche Messeinheit (1) temperiert oder für die Messungen auf einer angestrebten Zieltemperatur gehalten wird.

8. Verfahren nach einem der Ansprüche 1 bis 7, **dadurch gekennzeichnet, dass** das Peltier-Element (5) bzw. die Peltier-Element-Kaskade an ihrer dem Behälter (2) abgewandten Rückfläche, vorzugsweise abhängig von der gewünschten Temperatureinstellung im Speicherblock (4), beheizt oder gekühlt wird.

9. Verfahren nach einem der Ansprüche 1 bis 8, **dadurch gekennzeichnet, dass** bei einer Abkühlung des Behälters (2) das Peltier-Element (3) auf volle Leistung geschaltet wird und kurz vor oder bei Erreichen der Zieltemperatur in einem Regelmodus zur Konstanthaltung der Temperatur des Behälters (2) geschaltet wird.

10. Temperierbehälter mit einem Probeaufnahmeraum (15) und mit zumindest einem unter Sicherstellung eines Wärmeübergangskontaktes an der Außenwandfläche des Behälters (2) angebrachten Peltier-Elementes (3), insbesondere zur Durchführung des Verfahrens nach einem der Ansprüche 1 bis 9, **dadurch gekennzeichnet,**
- **dass** an der dem Behälter (2) abgewandten Fläche des Peltier-Elementes (3) unter Sicherstellung eines Wärmeübergangskontaktes ein Speicherblock (4) aus metallischem Material angebracht ist,
- **dass** an der dem Peltier-Element (3) abgewandten Rückfläche des Speicherblockes (4) unter Sicherstellung eines Wärmeübergangskontaktes ein weiteres Peltier-Element (5) oder eine Peltier-Element-Kaskade angebracht ist,
- **dass** zur Veränderung des Temperaturniveaus des Behälters (2) auf eine angestrebte Zieltemperatur hin oder in Richtung dieser Zieltemperatur eine Steuereinheit (7) für den Stromdurchfluss durch das Peltier-Element (3) und das weitere Peltier-Element (5) oder die Peltier-Element-Kaskade vorgesehen ist, mit welcher Steuereinheit (7) der Speicherblock (4) mit dem weiteren Peltier-Element (5) oder der Peltier-Element-Kaskade in Richtung dieser Zieltemperatur oder darüber hinaus erwärmbar oder abkühlbar ist und
- **dass** die Steuereinheit (7) für die Realisierung einer raschen Temperaturänderung im Behälter (2) bzw. dessen Probenaufnahmeraum (15) die Stromdurchflussrichtung des Peltier-Elementes (3) derart schaltet, dass dieses den Behälter (2) in Richtung auf die angestrebte Zieltemperatur erwärmt oder abkühlt und damit gleichzeitig den Übergang der im Speicherblock (4) enthaltenen Wärme- oder Kältemenge auf den Behälter (2) zur Verstärkung der vom Peltier-Element (3) erfolgenden Erwärmung oder Abkühlung freigibt.

11. Temperierbehälter nach Anspruch 10, **dadurch gekennzeichnet, dass** die Steuereinheit (7) während des Aufheizens oder des Abkühlens des Speicherblockes (4) das Temperaturniveau im Behälter (2) mit dem Peltier-Element (3) eingeregelt und gegebenenfalls konstant hält oder kontinuierlich verändert.

12. Temperierbehälter nach Anspruche 10 oder 11, **dadurch gekennzeichnet, dass** die Kapazität des Speicherblockes (4) für die Speicherung von Wärme- oder Kältemengen größer bemessen ist als die für eine gewünschte Temperaturänderung des Behälters (2) in Zielrichtung erforderliche Wärme- oder Kältemenge.

13. Temperierbehälter nach einem der Ansprüche 10 bis 12, **dadurch gekennzeichnet, dass** das Peltier-Element (5) oder die Peltier-Element-Kaskade an ihrer behälterfernen Fläche mit einem Wärmetauscher (6) versehen sind, mit dem die Peltier-Elemente (5) oder die Peltier-Element-Kaskade erwärmbar oder abkühlbar ist.

14. Temperierbehälter nach einem der Ansprüche 10 bis 13, **dadurch gekennzeichnet, dass** im Inneren des Behälters (2) bzw. des Probenaufnahmeraumes (15) eine Temperaturmesseinheit (11) angeordnet ist, die an die Steuereinheit (7) angeschlossen ist.

15. Temperierbehälter nach einem der Ansprüche 10 bis 14, **dadurch gekennzeichnet, dass** in den Temperierbehälter (2) eine Messeinheit (1) zur Bestimmung von temperaturabhängigen Materialparametern, insbesondere der viskosen Eigenschaften von Proben, vorzugsweise von Erdölerzeugnissen, eingesetzt ist.

16. Verwendung eines Temperierbehälters nach einem der Ansprüche 10 bis 15 zur Temperierung einer oder zur Temperatureinstellung in einer Messeinheit (1) zur Bestimmung von temperaturabhängigen Materialparametern, insbesondere der viskosen Eigenschaften von Proben, vorzugsweise von Erdölerzeugnissen.
